Europäisches Patentamt

European Patent Office

Office européen des brevets

(11) Publication number: **0 213 691 B1**

# EUROPEAN PATENT SPECIFICATION

(45) Date of publication of patent specification: **22.07.92** (51) Int. Cl.⁵: **C02F 3/28**

(21) Application number: **86304054.9**

(22) Date of filing: **28.05.86**

(54) A bioconversion reactor.

(30) Priority: **03.09.85 US 771972**

(43) Date of publication of application:
**11.03.87 Bulletin 87/11**

(45) Publication of the grant of the patent:
**22.07.92 Bulletin 92/30**

(84) Designated Contracting States:
**DE FR GB**

(56) References cited:
EP-A- 0 013 538      EP-A- 0 145 792
WO-A-86/00293      CH-A- 253 466
NL-A- 256 235      US-A- 2 202 772

(73) Proprietor: **THE BOARD OF TRUSTEES OF THE LELAND STANFORD JUNIOR UNIVERSITY**
**Encina 105 Stanford University**
**Stanford California 94305(US)**

(72) Inventor: **McCarty, Perry L.**
**823 Sonoma Terrace**
**Stanford California 94305(US)**
Inventor: **Bachmann, Andre**
**Costerweg 19**
**6701 BH Wageningen(NL)**

(74) Representative: **Allard, Susan Joyce et al**
**BOULT, WADE & TENNANT, 27 Furnival Street**
**London EC4A 1PO(GB)**

## Description

The invention relates to the anaerobic fermentation of organic materials, and more particularly to a bioconversion reactor for use in the anaerobic fermentation of organic materials.

The microbiological conversion of organic materials to methane gas is thought to have great potential for satisfying a significant proportion of the current demand for natural gas in the United States. The technology associated with this process has progressed to the point where it can be commercialized and can produce cost-competitive energy in some instances. Fermentation to methane has an advantage in that it produces a high-quality, clean-product gas which can be readily transported by existing gas-distribution networks. Additionally, organic materials which are too wet for direct combustion can be fermented to methane. Another advantage is that the nutrient value of the biomass is not destroyed by methane fermentation and can be recycled back to the land.

Anaerobic fermentation is also becoming much more attractive for the treatment of many organic-containing industrial wastewaters as it produces rather than consumes energy, the problem with the more commonly used aerobic processes for treating wastewaters. Interest in the anaerobic fermentation of industrial wastewaters has been strong; however, the availability of a reliable process that operates efficiently at short detention times has been lacking.

A typical anaerobic fermentation process involves flowing a liquid stream, which contains the organic materials to be treated, into a reactor which has been seeded with a biomass or microorganisms. The process may or may not involve pretreatment of the organic materials before anaerobic fermentation of the resulting soluble and particulate materials in the reactor. For the more dilute organic-material streams (less than 2% organics), the fermentation reactor must be a solid contact type in which the residence time of the microorganisms in the reactor is much greater than that of the liquid stream or effluent in the system. Among the problems with present reactor designs is that it is difficult to maintain a long residence time for the microorganisms in the reactor, either because they cannot be held within the reactor, or if they are, the resulting high concentrations tend to clog the reactor.

Several systems have been developed in the past. The simplest and least expensive system comprises an upflow unit with no media or mixing in the reactor, but with a separation funnel at the top to permit separation of gas from the liquid and biological solids within the reactor.

This design is described in an article by Lettinga et al., "Anaerobic Treatment of Methanolic Wastes", Water Research, Vol. 13, pp. 725-737, 1979. This system depends on the ability of bacterial solids to flocculate and remain for the most part in the lower zone of the reactor. Mixing of entering waste and bacterial solids is encouraged primarily by the turbulence created by the rising gas bubbles. This process is termed the upflow anaerobic sludge blanket process. The system is relatively inexpensive; however, its reliability is variable due to the difficulty in controlling the bacterial solids. Although the reactor has operated at less than a one-day detention time, gas-induced rising of bacterial solids and clogging of exit ports have been problems.

A second system is the anaerobic filter, which is described in an article by Young and McCarty, "The Anaerobic Filter For Waste Treatment", Journal Water Pollution Control Federation, Vol. 41, R160-173, May 1969. This may be an upflow or downflow system in which a fixed media such as rock is used to keep the bacteria within the system. The system can operate at a one-day or less detention time and is very reliable. However, the media, the rocks, occupy a lot of space, making the reactor very large. Other media such as plastics could be used to reduce this problem, but are generally expensive. The reactor also can eventually become clogged, although some solutions to this problem have been claimed.

Another system is the fluidized bed reactor described in an article by Switzenbaum and Jewell, "Anaerobic Attached-Film Expanded-Bed Reactor Treatment", Journal Water Pollution Control Federation, Vol. 52, pp. 1953-1965, July, 1980. This reactor is an upflow system which contains fine aluminum oxide particles or other media upon which the microorganisms attach and grow. Recycle is employed to obtain sufficient flow to fluidize or expand the media.

With this system contact between the organic-material stream and bacteria is optimized and detention times can be significantly reduced. However, this system is more difficult to operate reliably than other systems, especially on smaller scales. There is also some question about energy requirements for the high recycle rate required for media expansion.

Yet another system is the rotating biological contactor. Here, the organic materials or waste moves in a horizontal fashion around a series of slowly rotating plastic disks. The microorganisms are attached to the surfaces of the plastic disks. This system has a void volume of about 70%. This system seems to represent a good compromise between the fluidized bed reactor and the filter systems. However, the equipment is expensive and fairly complicated because of the rotating disk. This system is described in an article by Tait and Friedman, "Anaerobic Rotating Biological Contactor for Carbonaceous Waste Waters", Journal Water Pollution Control Federation, Vol. 52, pp. 2257-2269, 1980.

CH-A-253466 discloses a process for the continuous cultivation of microorganisms which in one embodiment makes use of a fermentation vessel in which are located verticle subdivisions arranged such that they form upflow and downflow chambers in which the height of the liquid decreases in the direction of the outlet pipe.

EP-A-0145792 discloses a process for the anaerobic digestion of organic waste which is in the form of a suspension. The process makes use of a reactor which is divided into a series of upflow and downflow chambers of differing sizes. It is an essential requirement of the reactor that the first chamber, a downflow chamber, be substantially larger than the subsequent upflow chamber.

EP-A-0013538 discloses a reactor for use in the recovery of methane gas from organic sludge. The reactor contains baffles to divide the interior into a series of upflow and downflow chambers and discloses a cylindrical form.

The present invention is a continuous bioconversion reactor which encompasses some of the advantages of the above reactors without their disadvantages. The system uses a series of baffles wherein the liquid stream containing the organic materials flows under and over the baffles as it passes from the inlet port to the outlet port of the reactor. The bacteria or microorganisms tend to rise and fall within the reactor but move horizontally at a slow rate. The reactor is simple in design, reliable, and serves to maintain the microorganisms within the reactor so that treatment detention times of one day or less are possible.

We have now developed an improved bioconversion reactor for the anaerobic fermentation of organic materials, which is simple in design, reliable, and which maintains the microorganisms within the reactor so that treatment detention times of the organic materials of one-day or less are possible.

The present invention provides a bioconversion reactor for the anaerobic fermentation of organic material contained in a liquid stream, comprising: a shell having top, bottom and side walls enclosing a predetermined volume; an inlet port in the shell through which the liquid stream enters the shell; an outlet port in the shell through which the liquid stream exits the shell; a plurality of baffles located within the shell and spaced from one another and positioned with the sides thereof joined to the side walls of the shell, at least one of the baffles being joined at the lower edge thereof to the bottom wall of the shell to extend substantially vertically upwardly therefrom and the lower edge of the other of the baffles being spaced from the bottom wall to define a flow passageway for the flow of the liquid stream therethrough, the baffles dividing the shell into upflow and downflow chambers; an upflow chamber for the flow of the liquid stream therethrough in fluid communication with one side of the flow passageway; a downflow chamber for the flow of the liquid stream therethrough in fluid communication with the other side of the flow passageway, characterised in that the volume of the downflow chamber is less than that of the upflow chamber; wherein, in use, the liquid stream flows downwardly through the downflow chamber and then through the flow passageway to flow upwardly through the upflow chamber, the baffles impeding the flow of microorganisms through the shell so that the microorganisms remain therein as the liquid stream flows therethrough.

The bioconversion reactor of the present invention will be described in more detail hereinafter in conjunction with the drawings wherein:

Figure 1 is a schematic diagram illustrating a bioconversion reactor, representative of the present invention with the exception that the upflow and downflow chambers have approximately equal volumes;

Figure 2 is a schematic diagram which illustrates an embodiment of the bioconversion reactor of the present invention;

Figure 3 is a schematic diagram illustrating a bioconversion reactor, representative of the present invention with the exception that the upflow and downflow chambers have approximately equal volumes;

Figure 4 schematically illustrates an embodiment of the bioconversion reactor of the present invention wherein the reactor is cylindrical in shape. Figures 4A and 4B are respresentative of the present invention with the exception that the upflow and downflow chambers have approximately equal volumes.

Figure 5 is a schematic diagram which illustrated still another embodiment of the bioconversion reactor of the present invention; and

Figures 6 and 7 are schematic diagrams which illustrate a further embodiment of the present invention.

The bioconversion reactor of the present invention has been developed to anaerobically ferment organic materials. For instance, the reactor may be used to anaerobically ferment organic-containing streams from processing agricultural residues such as corn stover, wheat straw and rice straw for the production of methane gas. The reactor may also be used in the anaerobic treatment of industrial wastewaters. A typical anaerobic treatment process may or may not involve pretreatment of the organic materials before anaerobic fermentation of the resulting soluble and particulate matter. To treat relatively dilute organic wastes (less than 2% organic material), the residence time of the microorganisms in the reactor must be much greater than that of the liquid stream in which the organic materials are contained. The bioconversion reactor of the present invention is suitable for use in the anaerobic treatment of very dilute to medium strength organic streams.

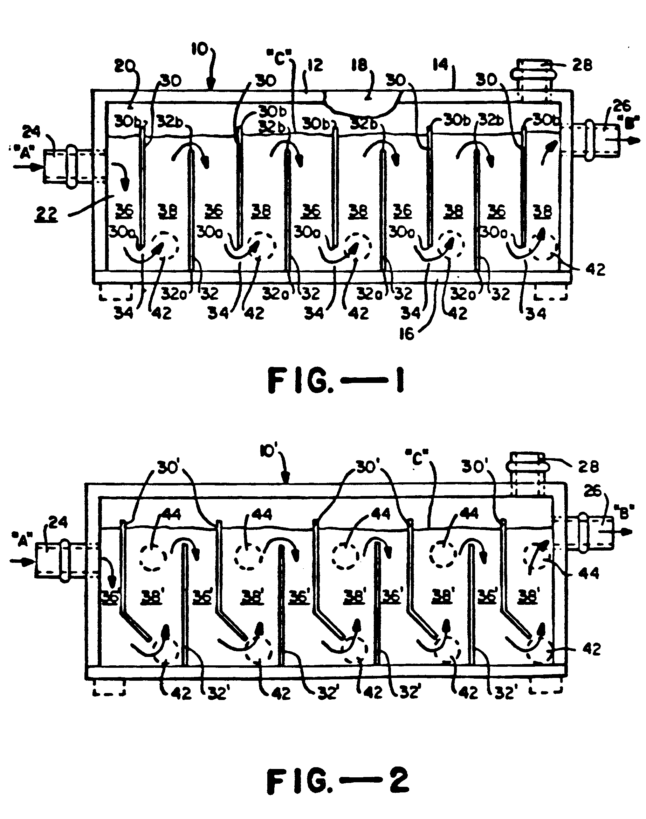

Referring to the drawings, wherein like components are designated by like reference numerals in the various figures, attention is first directed to Figure 1.

Bioconversion reactor 10 (Fig.1) comprises a shell 12 which is rectangular in shape and which has top, bottom and side walls 14, 16, 18, and 20, respectively, defining a predetermined volume 22. Reactor 10 has an inlet port 24 and an outlet port 26. The liquid stream in which the organic materials are contained enters the interior of shell 12 or predetermined volume 22 through inlet port 24. The effluent exits the reactor through outlet port 26. The direction of the flow of the stream through the reactor is indicated by arrows "A" and "B". Recycle of the effluent stream to mix with and dilute the influent stream may or may not be used. A gas port 28 in shell 12 may also be provided. Methane and other gases produced by the anaerobic fermentation of the organic materials within shell 12 can escape therefrom through gas port 28.

A plurality of baffles 30 and 32 are positioned within predetermined volume 22, along the length of shell 12. Baffles 30 and 32 are rectangular in shape and are affixed on each side thereof to side walls 18 and 20 of shell 12. The baffles therefore extend across the cross-sectional dimension of the shell. Baffles 32 are affixed at the lower edges thereof 32a to bottom wall 16 of the shell to be substantially perpendicular therewith. The lower edges 30a of baffles 30 are spaced from bottom wall 16 to define flow passageways 34 therebetween. Baffles 30 and 32 are substantially parallel to one another. Should shell 12 be other than rectangular in shape, for example square or cylindrical, baffles 30 and 32 will be configured accordingly.

Baffles 30 and 32 divide the interior of shell 12 into a plurality of downflow and upflow chambers 36 and 38, respectively. The cross-sectional dimensions of the downflow and upflow chambers are substantially equal. The downflow chambers 36 ore in fluid communication with fluid passageways 34 on that side of the passageways nearest inlet port 24. The upflow chambers 38 are in fluid communication with that side of flow passageways 34 nearest outlet port 26. The level of the liquid stream within shell 12 is represented by line "C".

The upper edges 30b of baffles 30 extend beyond this level. The construction of bioconversion reactor 10 permits a high volume of liquid in chambers 36 and 38 relative to predetermined volume 22, thus giving efficiency of reactor volume usage. The large open spaces comprising chambers 36 and 38 and the fluid passageways 34 between the chambers essentially eliminate clogging of the reactor by the microorganisms or biomass.

The bioconversion reactor 10 may also include a plurality of sample ports 42 spaced at intervals along the longitudinal dimension of the reactor. The sampling ports facilitate sampling of the liquid stream in the reactor and permit wasting of solids should the reactor clog.

As is well-known in the art, reactor 10 is seeded with microorganisms which react with the organic material in the liquid stream flowing through the reactor to anaerobically ferment those materials. The flow through the reactor is along the flow path established by vertical baffles 30 and 32. The baffles force the liquid stream to flow under and over them as it passes from the inlet to the outlet port. More particularly, the liquid stream entering reactor 10 flows downwardly through downflow chamber 36, around the lower edge 30a of baffle 30, that is, through flow passageway 34, and then upwardly through upflow chamber 38. From upflow chamber 38, the liquid stream flows around the upper edge 32b of baffle 32 to again flow in a downward direction through the next respective downflow chamber 36. The flow pattern is repeated as the liquid stream passes from inlet port 24 to outlet port 26. (see the unmarked, U-shaped arrows). The baffles present a barrier to the microorganisms or bacteria in the reactor which impedes their flow through the reactor. The microorganisms therefore tend to rise and fall within the reactor but move horizontally only at a very slow rate. This maintains the microorganisms within the reactor as the liquid stream passes therethrough so that treatment detention times of one day or less are possible.

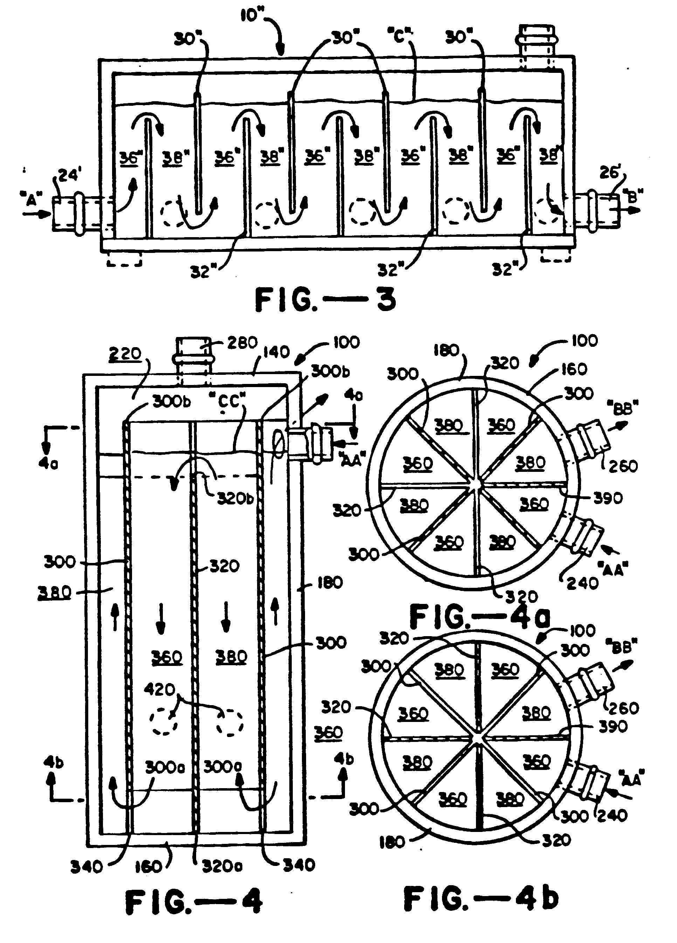

An embodiment of the present invention bioconversion reactor 10', is illustrated in Figure 2. Bioconversion reactor 10' is similar to reactor 10, differing in the configuration of baffles 30 and the relative sizes of the upflow and downflow chambers. In reactor 10', downflow chambers 36' have a smaller cross-sectional dimension and hence volume than upflow chambers 38'. Most of the microorganisms in the reactor were found to collect in the upflow chambers. Therefore, to promote mixing of the organic materials and the microorganisms and thereby increase the efficiency of the reactor, the downflow chambers were narrowed and the upflow chambers widened. In addition, the lower edges 30a of baffles 30 were slanted in the direction of upflow chambers 38' and thus away from the direction of the flow through the reactor. This was done to route a greater volume of flow to the center of upflow chambers 36' to further enhance mixing of the microorganisms and organic materials. The number of sample ports in reactor 10' was also increased by adding a second row of ports 44 to facilitate sampling and wasting of solids should clogging occur.

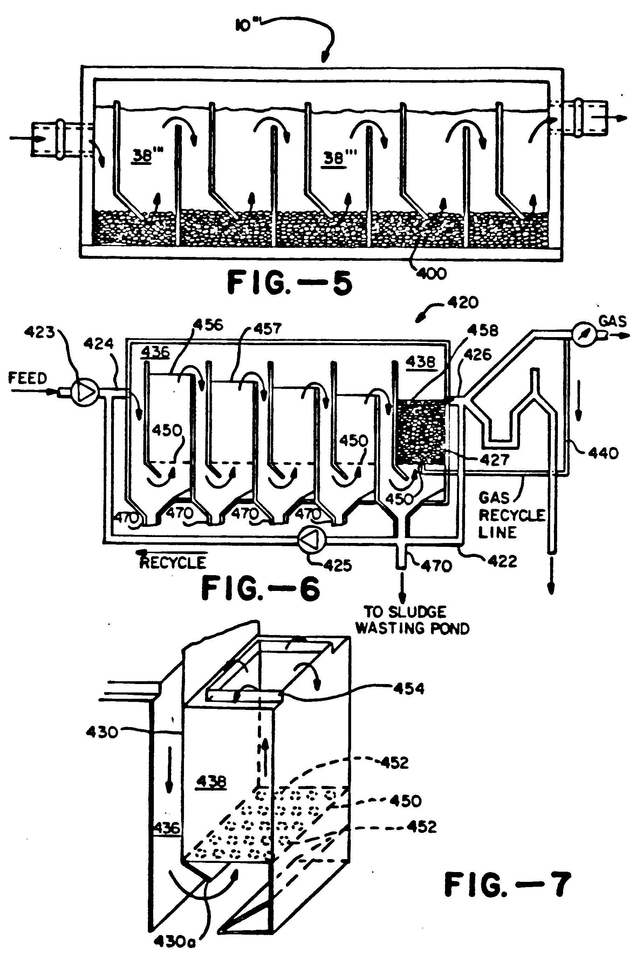

Figure 3 shows bioconversion reactor 10" which is similar to reactors 10 and 10'. The relative positions of the upflow and downflow chambers and the inlet and outlet ports, however, have been changed. Particularly, baffles 30" and 32" have been repositioned within the reactor so that an upflow chamber 36" is located adjacent to inlet port 24', which has been moved to a position near the bottom wall of the reactor. Similarly, outlet port 26' has been lowered, and a downflow chamber 38" has been located adjacent to outlet port 26'. Another embodiment could have a downflow chamber adjacent to both the inlet port (see Figure 1) and the outlet port (see Figure 3). The baffles could also be positioned such that upflow chambers are located adjacent to the inlet and outlet ports. The embodiment of Figure 2 could be modified in the same manner.

Bioconversion reactor 100 shown in Figure 4 is another embodiment. Figures 4A and 4B are representative of the present invention with the exception that the upflow and downflow chambers have approximately equal volumes. Reactor 100 is cylindrical in shape with bottom wall 160 of its shell 120 forming the base of the reactor. The reactor has inlet and outlet ports 240 and 260, respectively. The direction of the stream flow is indicated by arrows "AA", "BB", and the unmarked arrows within predetermined volume 220, which is defined by the top, bottom and circumferential walls 140, 160 and 180, respectively, of shell 120. A gas port 280 and sample ports 420 are also provided.

A plurality of vertical baffles 300 and 320 are positioned within predetermined volume 220 of cylindrical shell 100 to extend radially from the central axis thereof. The baffles are rectangular in shape and are joined on one side thereof to the interior of circumferential wall 180. The baffles are vertical and thus parallel to the shell's central axis. Baffles 320 are joined at the lower edges 320a thereof to bottom wall 160 of the shell. The lower edges 300a of baffles 300 are spaced from the bottom wall 160 to define flow passageways 340 therebetween.

Baffles 300 and 320 divide the interior of shell 120 into a plurality of downflow and upflow chambers 360 and 380, respectively, which are shaped like pie slices (see Figures 4A and 4B). The present invention determines that the volume of the downflow chamber is less than that of the upflow chamber. The lower edges 300a may be straight as shown in Figure 4 or slanted in the direction of the upflow chamber as in Figure 2.

The level of the stream in the reactor is represented by line "CC". The upper edges 300b of baffles 300 may extend beyond this level as shown. The flow through the reactor is along the flow path established by baffles 300 and 320. The baffles force the liquid to flow under and over them as it passes from the inlet to the outlet port. The liquid stream entering reactor 100 through inlet port 240 flows downwardly through downflow chamber 360, around the lower edge 300a of baffle 300, that is, through flow passageway 340, and then upwardly through upflow chamber 380. From upflow chamber 380, the liquid stream flows around the upper edge 320b of baffle 320 to again flow in a downward direction through the next downflow chamber. The flow pattern is repeated as the liquid stream flows around the cylindrical shell from the inlet port to the outlet port. A wall 390 blocks fluid communication between the chambers adjacent to the inlet and outlet ports so that the stream will exit through the outlet port. The baffles present a barrier to the microorganisms which impede their flow through the reactor. The arrangement of the baffles, and inlet and outlet ports may also be modified as discussed in connection with the bioconversion reactor of Figure 3.

Another embodiment of the present invention is shown in Figure 5. This embodiment is similar to the reactor shown in Figure 2. Reactor 10''' further includes media 400 such as stones or plastic shapes such as spheres, saddles, or other open porous forms having density greater than water are placed in the bottom portions of each chamber. This media may have an average outside dimension of 2.5 to 10 cm (1 to 4 inches). The purpose is in part to achieve better distribution of liquid so that it flows more uniformly up through the upflow chambers 38''', thereby achieving better treatment and less short circuiting. In addition, the bateria within the chambers are less likely to flow out of the system, especially when handling dilute soluble wastewaters with organic content measured at the chemical oxygen demand of from 200 to 4000 mg/l. With wastewater this dilute, flow rates tend to be relatively fast such that bacteria have a greater tendency to be transported out of the system. The media prevents this from occurring. This modification combines some of the advantages of an anaerobic filter system without the disadvantage of the high costs and greater clogging potential that results when the entire reactor contains such media.

Yet another embodiment is illustrated in Figures 6 and 7. Reactor 420 of this embodiment includes means for recycling effluent to mix with the influent. This is desirable to reduce the potential for low-pH conditions in the first chambers, and for effecting a better distribution of bacterial species throughout the reactor, especially with more concentrated wastewaters.

The recycle system may include suitable conduit 422 for recycling effluent from the outlet port 426 to the inlet port 424 where it enters the reactor at the first downflow chamber 436. Pumps 423 and 425 are provided to control recycling and the introduction of influent.

The last upflow chamber 438 of reactor 420 incorporates suitable media 427, such as a plastic filling material, so that this chamber functions as a clarifying chamber to keep bacteria within the system. The effluent of the reactor is taken from the center of the last chamber by means of the piping illustrated.

A gas recycle line 440 is also included. The gas recycle enables suspended solids to be loosened in order to prevent clogging of the filling material.

Each chamber includes a system that permits solids collection and wasting. Piping 470 at the bottom of each chamber represents such a system. The associated valving and pumps for this system are not shown.

Each upflow chamber 438 also includes a false bottom 450 (see Figure 7) having a series of orifices 452 through which the fluid from the downflow chamber enters the upflow chamber. The false bottom provides enhanced distribution of liquids as it enters the upflow chamber, thus providing more uniform upward flow of liquid. As shown, the lower edges 430a of baffles 430 are slanted in the direction of the upflow chambers.

Overflow rims 454 may also be included on the upflow chambers. Such rims help keep the biomass within the upflow chambers by encouraging more uniform flow of liquid.

Further, as can be best seen in Figure 6, the chambers of reactor 420 have a step-down configuration wherein the overflow level 456 in the first upflow chamber is the highest with the overflow level 458 in the last chamber being the lowest. The overflow levels between adjacent upflow chambers may differ in height from about 3 to 5 centimeters. As such, overflow level 456 may be about 3 to 5 centimeters higher than overflow level 457.

Although certain specific embodiments of the invention have been described herein in detail, the invention is not to be limited to only such embodiments, but rather only by the appended claims.

## Claims

1. A bioconversion reactor for the anaerobic fermentation of organic material contained in a liquid stream, comprising: a shell having top, bottom and side walls enclosing a predetermined volume; an inlet port in the shell through which the liquid stream enters the shell; an outlet port in the shell through which the liquid stream exits the shell; a plurality of baffles located within the shell and spaced from one another and positioned with the sides thereof joined to the side walls of the shell, at least one of the baffles being joined at the lower edge thereof to the bottom wall of the shell to extend substantially vertically upwardly therefrom and the lower edge of the other of the baffles being spaced from the bottom wall to define a flow passageway for the flow of the liquid stream therethrough, the baffles dividing the shell into upflow and downflow chambers; an upflow chamber for the flow of the liquid stream therethrough in fluid communication with one side of the flow passageway; a downflow chamber for the flow of the liquid stream therethrough in fluid communication with the other side of the flow passageway, characterised in that the volume of the downflow chamber is less than that of the upflow chamber; wherein, in use, the liquid stream flows downwardly through the downflow chamber and then through the flow passageway to flow upwardly through the upflow chamber, the baffles impeding the flow of microorganisms through the shell so that the microorganisms remain therein as the liquid stream flows therethrough.

2. A bioconversion reactor as claimed in claim 1 wherein the baffles are located along the length of the shell in a substantially parallel manner.

3. A bioconversion reactor as claimed in claim 1 wherein the shell is cylindrical and the baffles are located such that they radiate from the central axis of the shell and each outer side thereof is joined to the circumferential wall of the shell.

4. A bioconversion reactor as claimed in any one of the preceding claims wherein the lower edge of the other of the baffles is slanted away from the downflow chamber.

5. A bioconversion reactor as claimed in any one of the preceding claims wherein the baffle closest to the inlet port has its lower edge thereof joined to the bottom wall of the shell.

6. A bioconversion reactor as claimed in claim 5 wherein the baffle closest to the outlet port has its lower edge thereof joined to the bottom wall of the shell.

7. A bioconversion reactor as claimed in any one of the preceding claims wherein the upflow chamber nearest the outlet port has media therein so that the downflow chamber functions as a clarifying chamber.

8. A bioconversion reactor as claimed in any one of the preceding claims further including media located at the bottom of the shell between the baffles for achieving a more uniform flow of the liquid stream through the predetermined volume.

9. A bioconversion reactor as claimed in any one of the preceding claims further including means for recycling the liquid stream leaving the shell so that it may be mixed with the liquid stream entering the shell.

10. A bioconversion reactor as claimed in any one of the preceding claims further including a gas recycle line for recycling gas from the outlet port back into the shell.

11. A bioconversion reactor as claimed in any one of the preceding claims further including means for collecting and wasting solids in the shell.

12. A bioconversion reactor as claimed in any one of the preceding claims wherein the upflow chamber includes means for providing an enhanced distribution of flow of the liquid stream entering therein.

13. A bioconversion reactor as claimed in claim 12 wherein the enhanced distribution means includes a false bottom at the bottom of the upflow chamber, the false bottom having a series of orifices formed therein.

14. A bioconversion reactor as claimed in claim 12 wherein the enhanced distribution means includes a partition located at the bottom of the upflow chamber, the partition having a number of fluid passageways provided therewith.

15. A bioconversion reactor as claimed in claim 14 wherein an overflow rim is located at the top of the upflow chamber.

16. A bioconversion reactor as claimed in any one of the preceding claims wherein the overflow levels in successive upflow chambers differ in height.

17. A bioconversion reactor as claimed in claim 16 wherein the overflow level of the upflow chambers decrease in height away from the inlet port.

18. A bioconversion reactor as claimed in claim 17 wherein the overflow levels of the upflow chambers have a step-down configuration.

19. A bioconversion reactor as claimed in claim 18 wherein the respective overflow levels of adjacent upflow chambers decrease in height with the highest overflow level located nearest to the inlet port.

20. A bioconversion reactor as claimed in claim 19 wherein the decrease in height is between about 2 to 5 centimeters between adjacent upflow chambers.

21. A bioconversion reactor as claimed in any one of the preceding claims including a gas port through which gases produced by the anaerobic fermentation of organic material in the shell may pass.

**Revendications**

1. Réacteur de bioconversion pour la fermentation anaérobie de matières organiques contenues dans un courant liquide, qui comprend : une cuve possédant des parois supérieure, inférieure et latérales enfermant un volume prédéterminé ; un orifice d'entrée prévu dans la cuve, à travers lequel le courant de liquide pénètre dans la cuve ; un orifice de sortie prévu dans la cuve, à travers lequel le courant de liquide sort de la cuve ; une pluralité de chicanes placées dans la cuve, espacées l'une de l'autre et placées de manière que leurs côtés soient réunis aux parois latérales de la cuve, au moins une des chicanes étant réunie à la paroi inférieure de la cuve au niveau de son bord inférieur pour s'étendre sensiblement verticalement vers le haut à partir de ce bord et le bord inférieur de l'autre des chicanes étant espacé de la paroi inférieure pour définir un passage d'écoulement qui donne passage à l'écoulement du courant de liquide, les chicanes divisant la cuve en chambres d'écoulement ascendant et chambres d'écoulement descendant ; une chambre d'écoulement ascendant destinée à se laisser traverser Par le courant de liquide étant en communication fluidique avec un côté du passage d'écoulement ; une chambre d'écoulement descendant destinée à se laisser traverser par le courant de liquide étant en communication fluidique avec l'autre côté du passage d'écoulement, caractérisé en ce que le volume de la chambre d'écoulement descendant est inférieur à celui de la chambre d'écoulement ascendant ; cependant qu'en utilisation, le courant de liquide s'écoule vers le bas dans la chambre d'écoulement descendant puis traverse le passage d'écoulement pour s'écouler de bas en haut dans la chambre d'écoulement ascendant, les chicanes empêchant les micro-organismes de circuler dans la cuve, de sorte que les micro-organismes restent dans cette cuve pendant que le courant de liquide circule à travers la cuve.

2. Réacteur de bioconversion selon la revendication 1, dans lequel les chicanes sont réparties sur la longueur de la cuve, dans des disposition sensiblement parallèles.

3. Réacteur de bioconversion selon la revendication 1, dans lequel la cuve est cylindrique et les chicanes sont disposées de manière à rayonner à partir de l'axe central de la cuve, et le côté extérieur de chacune de ces chicanes est réuni à la paroi circonférentielle de la cuve.

4. Réacteur de bioconversion selon une quelconque des revendications précédentes, dans lequel le bord inférieur de ladite autre chicane est incliné dans le sens qui s'éloigne de la chambre d'écoulement descendant.

5. Réacteur de bioconversion selon l'une quelconque des revendications précédentes, dans lequel la chicane la plus proche de l'orifice d'entrée a son bord inférieur réuni à la paroi inférieure de la cuve.

6. Réacteur de bioconversion selon la revendication 5, dans lequel la chicane la plus proche de l'orifice de sortie a son bord inférieur réuni à la paroi inférieure de la cuve.

7. Réacteur de bioconversion selon l'une quelconque des revendications précédentes, dans lequel la chambre d'écoulement ascendant la plus proche de l'orifice de sortie présente intérieurement des moyens tels que la chambre d'écoulement descendant se comporte comme une chambre de clarification.

8. Réacteur de bioconversion selon l'une quelconque des revendications précédentes, comprenant en outre des milieux placés au fond de la cuve, entre les chicanes, pour assurer un écoulement plus uniforme du courant de liquide dans le volume prédéterminé.

9. Réacteur de bioconversion selon l'une quelconque des revendications précédentes, comprenant en outre des moyens servant à recycler le courant de liquide qui sort de la cuve de manière qu'il puisse être mélangé au courant de liquide qui pénètre dans la cuve.

10. Réacteur de bioconversion selon l'une quelconque des revendications précédentes, comprenant en outre une conduite de recyclage de gaz servant à recycler les gaz de l'orifice d'entrée vers la cuve.

11. Réacteur de bioconversion selon l'une quelconque des revendications précédentes, comprenant en outre des moyens servant à recueillir et évacuer les solides contenus dans la cuve.

12. Réacteur de bioconversion selon l'une quelconque des revendications précédentes, dans lequel la chambre d'écoulement ascendant comprend des moyens permettant d'assurer une distribution renforcée de l'écoulement du courant de liquide qui y pénètre.

13. Réacteur de bioconversion selon la revendication 12, dans lequel les moyens de distribution renforcée comprennent un faux fond prévu à la partie basse de la chambre d'écoulement ascendant, le faux fond présentant une série d'ouvertures qui y sont formés.

14. Réacteur de bioconversion selon la revendication 12, dans lequel les moyens de distribution renforcée comprennent une cloison placée à la base de la chambre d'écoulement ascendant, la cloison présentant un certain nombre de passages de fluide qui y sont prévus.

15. Réacteur de bioconversion selon la revendication 14, dans lequel un rebord de trop-plein est placé à la partie haute de la chambre d'écoulement ascendant.

16. Réacteur de bioconversion selon l'une quelconque des revendications précédentes, dans lequel les niveaux de trop-plein des chambres d'écoulement ascendant successives varient en hauteur.

17. Réacteur de bioconversion selon la revendication 16, dans lequel le niveau de trop-plein des chambres d'écoulement ascendant décroît en hauteur dans le sens qui s'éloigne de l'orifice d'entrée.

18. Réacteur de bioconversion selon la revendication 17, dans lequel les niveaux de trop-plein des chambres d'écoulement ascendant ont une configuration en escalier descendant.

19. Réacteur de bioconversion selon la revendication 18, dans lequel les niveaux de trop-plein respectif des chambres d'écoulement ascendant adjacentes décroîssent en hauteur, le niveau d'écoulement le plus élevé étant le plus rapproché de l'orifice d'entrée.

20. Réacteur de bioconversion selon la revendication 19, dans lequel la diminution de hauteur entre les chambres d'écoulement ascendant adjacentes est d'environ 2 à 5 centimètres.

21. Réacteur de bioconversion selon l'une quelconque des revendications précédentes, comprenant un orifice de gaz à travers lequel peuvent passer les gaz produits par la fermentation anaérobie de la matière organique contenue dans la cuve.

**Patentansprüche**

1. Bioumwandlungsreaktor für die anaerobe Fermentierung von in einem Flüssigkeitsstrom enthaltenem organischen Material
   - mit einem Mantel, der obere, untere und Seitenwände aufweist, welche ein vorbestimmtes Volumen einschließen;
   - mit einer Einlaßöffnung in dem Mantel, durch die der Flüssigkeitsstrom in den Mantel eintritt;
   - mit einer Auslaßöffnung in dem Mantel, durch die der Flüssigkeitsstrom aus dem Mantel austritt;
   - mit einer Vielzahl von Leitwänden, die sich innerhalb des Mantels befinden und im Abstand voneinander angeordnet und so positioniert sind, daß ihre Seiten mit den Seitenwänden des Mantels verbunden sind, wobei mindestens eine der Leitwände an ihrem unteren Rand so mit der unteren Wand des Mantels verbunden ist, daß sie sich von dieser im wesentlichen vertikal nach oben erstreckt, und der untere Rand der anderen der Leitwände so im Abstand von der unteren Wand angeordnet ist, daß sie einen Strömungskanal für den Durchgang des Flüssigkeitsstroms durch ihn hindurch begrenzt, wobei die Leitwände den Mantel in Aufwärtsstrom- und Abwärtsstromkammern teilen;
   - mit einer Aufwärtsstromkammer für den Durchgang des Flüssigkeitsstroms durch sie hindurch in Fluidverbindung mit einer Seite des Strömungskanals;
   - mit einer Abwärtsstromkammer für den Durchgang des Flüssigkeitsstroms durch sie hindurch in Fluidverbindung mit der anderen Seite des Strömungskanals,
   dadurch gekennzeichnet, daß das Volumen der Abwärtsstromkammer kleiner als das der Aufwärtsstromkammer ist, wobei im Einsatz der Flüssigkeitsstrom nach unten durch die Abwärtsstromkammer und dann durch den Strömungskanal fließt, um durch die Aufwärtsstromkammer nach oben zu fließen, wobei die Leitwände den Fluß von Mikroorganismen durch den Mantel so unterbinden, daß die Mikroorganismen in ihr zurückbleiben, wenn der Flüssigkeitsstrom durch sie hindurch fließt.

2. Bioumwandlungsreaktor nach Anspruch 1, bei welchem die Leitwände entlang der Länge des Mantels im wesentlichen parallel angeordnet sind.

3. Bioumwandlungsreaktor nach Anspruch 1, bei welchem der Mantel zylindrisch ist und die Leitwände so angeordnet sind, daß sie sich von der Mittelachse des Mantels radial ausbreiten und jede äußere Seite von ihnen mit der Umfangswand des Mantels verbunden ist.

4. Bioumwandlungsreaktor nach einem der vorhergehenden Ansprüche, bei welchem der untere Rand der anderen der Leitwände von der Abwärtsstromkammer weg geneigt ist.

5. Bioumwandlungsreaktor nach einem der vorhergehenden Ansprüche, bei welchem der untere Rand der der Einlaßöffnung am nächsten gelegenen Leitwand mit der unteren Wand des Mantels verbunden ist.

6. Bioumwandlungsreaktor nach Anspruch 5, bei welchem der unter Rand der der Auslaßöffnung am nächsten gelegenen Leitwand mit der unteren Wand des Mantels verbunden ist.

7. Bioumwandlungsreaktor nach einem der vorhergehenden Ansprüche, bei welchem die der Auslaßöffnung am nächsten gelegene Aufwärtsstromkammer Medien enthält, so daß die Abwärtsstromkammer als Klärkammer wirkt.

8. Bioumwandlungsreaktor nach einem der vorhergehenden Ansprüche, welcher ferner zur Erzielung eines gleichförmigeren Durchgangs des Flüssigkeitsstroms durch das vorherbestimmte Volumen Medien aufweist, die sich auf dem Boden des Mantels zwischen den Leitwänden befinden.

9. Bioumwandlungsreaktor nach einem der vorhergehenden Ansprüche, welcher ferner eine Einrichtung zum Rückführen des den Mantel verlassenden Flüssigkeitsstroms aufweist, so daß er mit dem in den Mantel eintretenden Flüssigkeitsstrom vermischt werden kann.

10. Bioumwandlungsreaktor nach einem der vorhergehenden Ansprüche, welcher ferner eine Gasrückführleitung zum Rückführen von Gas von der Auslaßöffnung zurück in den Mantel aufweist.

11. Bioumwandlungsreaktor nach einem der vorhergehenden Ansprüche, welcher ferner eine Einrichtung zum Sammeln und Ausscheiden von Feststoffen in dem Mantel aufweist.

12. Bioumwandlungsreaktor nach einem der vorhergehenden Ansprüche, bei welchem die Aufwärtsstromkammer eine Einrichtung zur Bereitstellung einer erhöhten Durchgangsverteilung des in sie eintretenden Flüssigkeitsstroms hat.

13. Bioumwandlungsreaktor nach Anspruch 12, bei welchem die Einrichtung zur erhöhten Verteilung am Boden der Aufwärtsstromkammer einen Doppelboden aufweist, wobei der Doppelboden eine Reihe von in ihm ausgebildeten Öffnungen aufweist.

14. Bioumwandlungsreaktor nach Anspruch 12, bei welchem die Einrichtung zur erhöhten Verteilung eine sich an dem Boden der Aufwärtsstromkammer befindende Trennwand aufweist, wobei die Trennwand mit einer Anzahl von Fluidkanälen versehen ist.

15. Bioumwandlungsreaktor nach Anspruch 14, bei welchem sich auf der Oberseite der Aufwärtsstromkammer ein Überlaufrand befindet.

16. Bioumwandlungsreaktor nach einem der vorhergehenden Ansprüche, bei welchem die Überlaufniveaus in aufeinanderfolgenden Aufwärtsstromkammern sich in der Höhe unterscheiden.

17. Bioumwandlungsreaktor nach Anspruch 16, bei welchem das Überlaufniveau der Aufwärtsstromkammern von der Einlaßöffnung weg an Höhe abnimmt.

18. Bioumwandlungsreaktor nach Anspruch 17, bei welchem die Überlaufniveaus der Aufwärtsstromkammern eine abwärts gestufe Anordnung haben.

19. Bioumwandlungsreaktor nach Anspruch 18, bei welchem die jeweiligen Überlaufniveaus von angrenzenden Aufwärtsstromkammern an Höhe abnehmen, wobei das höchste Überlaufniveau der Einlaßöffnung am nächsten liegt.

20. Bioumwandlungsreaktor nach Anspruch 19, bei welchem die Abnahme an Höhe zwischen benachbarten Aufwärtsstromkammern zwischen etwa 2 und 5 Zentimetern liegt.

21. Bioumwandlungsreaktor nach einem der vorhergehenden Ansprüche, der eine Gasöffnung aufweist, durch die durch die anaerobe Fermentierung von organischem Material in dem Mantel erzeugte Gase hindurchgehen können.

FIG. — 1

FIG. — 2

FIG. — 3

FIG. — 4

FIG. — 4a

FIG. — 4b

FIG.—5

FIG.—6

TO SLUDGE
WASTING POND

FIG.—7